# DEMANDE DE BREVET EUROPEEN

(11) **EP 3 339 273 A1**
(43) Date de publication de la demande: **27.06.2018**
(21) Numéro de dépôt: 17306799.2
(22) Date de dépôt: 18.12.2017
(51) Int. Cl.: C07C 2/22, C07C 7/10

(54) **PROCÉDÉ DE NEUTRALISATION D'UN SYSTÈME CATALYTIQUE DE DIMÉRISATION D'OLÉFINES CONTENANT AU MOINS UN DÉRIVÉ HALOGÈNE**

(30) Priorité: 22.12.2016 FR 1663138
(71) Demandeur: IFP Energies nouvelles, 92852 Rueil-Malmaison Cedex (FR)
(72) Inventeur: OLIVIER-BOURBIGOU, Helene, 69230 SAINT GENIS-LAVAL (FR); HUGUES, Francois, 69390 VERNAISON (FR); PELLIER, Emmanuel, 69420 TUPIN SEMONS (FR)

(57) **Abrégé**

L'invention concerne un procédé de neutralisation d'un système catalytique d'oligomérisation d'oléfines, ledit système catalytique comprenant au moins un dérivé halogéné, caractérisé en ce que l'on met en contact l'effluent réactionnel avec au moins un composé nitrile.

## Description

La présente invention concerne un procédé de neutralisation d'un système catalytique d'oligomérisation d'oléfines, en particulier un procédé de dimérisation d'oléfines, ledit système catalytique comprenant au moins un dérivé halogéné.

### Art antérieur

La transformation des oléfines légères grâce à un catalyseur homogène à base d'un métal de transition, en particulier à base de nickel, associé à activateur halogéné, par exemple un chloroalkylaluminium est étudiée depuis les années 50. Cette recherche a conduit au développement et à la commercialisation de différents procédés.

Par exemple, des octènes ou des hexènes ou des nonènes sont produits respectivement par dimérisation de butènes ou oligomérisation du propylène par le procédé Dimersol™ d'Axens (Revue de l'Institut Français du Pétrole, Vol. 37, N°5, septembre-octobre 1982, p 639). Les octènes peuvent être transformés avec de bons rendements par réaction d'hydroformylation puis d'hydrogénation en isononanols. Ces alcools en C9 (c'est-à-dire comprenant 9 atomes de carbone) sont notamment utilisés pour la synthèse de plastifiants de type phtalates pour le PVC. Les hexènes ou les nonènes peuvent aussi être utilisés comme base pour carburant à très bon indice d'octane.

La mise au point de systèmes catalytiques capables de dimériser les oléfines passe par le choix du métal de transition et des ligands adaptés. Parmi les systèmes de catalyseurs existants, plusieurs systèmes catalytiques à base de nickel utilisant des ligands différents ont été développés. On peut citer notamment à titre d'exemples les complexes d'halogénures de π-allyl nickel phosphine avec les acides de Lewis, comme cela est décrit dans le brevet français FR1410430B, les complexes d'halogénures de nickel phosphine avec les acides de Lewis, comme cela est décrit dans le brevet US3485881A, les complexes de nickel avec les ligands imino-imidazole comme cela est décrit dans le brevet français FR2979836B et les carboxylates de nickel avec les halogénures d'hydrocarbylaluminium, comme cela est décrit dans le brevet USA3 321546A. Dans de tels procédés, il s'avère nécessaire de neutraliser le catalyseur en fin de réaction pour éviter que la réaction ne se poursuive de façon indésirable.

Dans le brevet FR2114114B, il est décrit un procédé de désactivation d'un catalyseur de dimérisation d'oléfines, contenant au moins un composé halogéné mettant en oeuvre un contact de l'effluent de réaction avec de l'ammoniac anhydre ou une amine.

Le brevet US 4642408B décrit un procédé de traitement d'un effluent d'une réaction d'oligomérisation d'oléfines en présence d'un catalyseur contenant un composé halogéné par traitement de l'effluent dans une première étape (1) par une solution d'ammoniac anhydre, puis dans une seconde étape (2) par lavage avec une solution aqueuse de métaux alcalins, avec la condition que l'effluent soit traité avant ou après les étapes (1) et (2) par de l'oxygène ou un gas contenant de l'oxygène.

Les procédés décrits dans l'art antérieur présentent une efficacité de neutralisation satisfaisante, mais posent le problème de la sécurité liée à l'utilisation de certains inhibiteurs tels que l'ammoniac ou les amines, notamment en raison de leur état physique gazeux et/ou de leur toxicité.

La demanderesse dans ses recherches a mis au point un nouveau procédé de neutralisation d'un système catalytique d'oligomérisation d'oléfines, de préférence de dimérisation d'oléfines, ledit système catalytique comprenant au moins un dérivé halogéné, mettant en oeuvre une étape de contact de l'effluent réactionnel avec au moins un composé nitrile.

Il a été constaté de manière surprenante qu'un tel procédé permettait une neutralisation efficace du système catalytique comprenant au moins un dérivé halogéné mis en oeuvre dans la réaction d'oligomérisation d'oléfines. En particulier, le procédé selon l'invention permet d'éviter la formation de produits organiques chlorés issus des réactions secondaires entre les dérivés halogénés présents dans le système catalytique et les oléfines formées lors de la réaction de dimérisation.

Un objectif du procédé de neutralisation selon l'invention est d'éviter que les oléfines formées soient contaminées par des quantités non négligeables d'halogènes provenant du système catalytique, notamment par la formation de produits organiques halogénés (chlorés ou bromés). Ces halogènes peuvent être particulièrement nuisibles et difficiles à éliminer des oléfines notamment dans le cas où les oléfines sont utilisées ultérieurement dans des réactions spécifiques comme une réaction d'hydroformylation ou d'hydrogénation.

### Description détaillée de l'invention

L'objet de l'invention concerne un procédé de neutralisation d'un système catalytique d'oligomérisation d'oléfines, de préférence de dimérisation d'oléfines, ledit système catalytique comprenant au moins un dérivé halogéné, caractérisé en ce que l'on met en contact l'effluent réactionnel comprenant ledit système catalytique avec au moins un composé nitrile RC=N comprenant au moins un groupement carbonitrile -C=N, R étant un groupe hydrocarbyle possédant de 1 à 20 atomes de carbone, saturé ou insaturé, linéaire ou ramifié, cyclique ou aromatique, comprenant ou non un hétéroatome.

Le procédé selon l'invention s'applique aux effluents réactionnels comprenant des dimères et des oligomères produits par des réactions de dimérisation ou d'oligomérisation d'oléfines mettant en oeuvre un système catalytique comprenant un dérivé halogéné. Avantageusement, ledit système catalytique est un système catalytique homogène en phase liquide.

L'oligomérisation est définie comme la transformation d'une unité monomère CₙH₂ₙ (n= 2 à 10) en un composé ou mélange de composés de formule générale CₚH₂ₚ avec 4 ≤ p ≤ 80, de préférence avec 4 ≤ p ≤ 50, de manière préférée avec 4 ≤ p ≤ 26 et de manière plus préférée avec 4 ≤ p ≤ 14.

L'effluent réactionnel est avantageusement issu d'un procédé d'oligomérisation, de préférence d'un procédé de dimérisation des oléfines opéré à une pression totale comprise entre la pression atmosphérique et 20 MPa, de préférence entre 0,5 et 8 MPa, et à une température comprise entre -40 et +250°C, de préférence entre -20°C et 150°C.

Les oléfines utilisées comme charge dans le procédé d'oligomérisation d'oléfines ou de préférence dans le procédé de dimérisation des oléfines sont des oléfines comportant de 2 à 10 atomes de carbone et de préférence lesdites oléfines sont choisies parmi l'éthylène, le propylène, les n-butènes et les n-pentènes, seuls ou en mélange, purs ou dilués, de préférence les oléfines utilisées sont les n-butènes, le propylène et/ou l'éthylène. Dans le cas où lesdites oléfines sont diluées, lesdites oléfines sont diluées par un ou plusieurs alcane(s), tels qu'on les trouve dans des « coupes » issues des procédés de raffinage du pétrole, comme le craquage catalytique ou le craquage à la vapeur. Lesdites oléfines peuvent venir de ressources non fossiles telles que la biomasse. Par exemple, les oléfines utilisées dans le procédé d'oligomérisation peuvent avantageusement être produites à partir d'alcools, et en particulier par déshydratation des alcools.

Le procédé d'oligomérisation d'oléfines, de préférence le procédé de dimérisation des oléfines peut être opéré en présence d'un solvant. Dans un tel cas, le solvant peut être choisi parmi les solvants organiques et les hydrocarbures saturés, insaturés, aromatiques ou non, cycliques ou non. En particulier, ledit solvant peut être choisi parmi l'hexane, le cyclohexane, le méthylecyclohexane, l'heptane, le butane ou l'isobutane, les monooléfines ou dioléfines comportant de préférence 4 à 20 atomes de carbone, le benzène, le toluène, l'ortho-xylène, le mésitylène, l'éthylbenzène, purs ou en mélange, et les liquides ioniques.

Le procédé de neutralisation selon l'invention s'applique à tout système catalytique d'oligomérisation ou de dimérisation d'oléfines mettant en oeuvre un catalyseur comprenant un dérivé halogéné. Selon le procédé de l'invention, le système catalytique, de préférence homogène comprend au moins un dérivé halogéné.

Le dérivé halogéné peut être tout composé halogéné connu de l'homme du métier dans les systèmes catalytiques d'oligomérisation des oléfines.

Avantageusement, le dérivé halogéné est un halogénure d'alkyl aluminium de formule AlR'ₘX₃₋ₘ dans laquelle R' est un radical hydrocarbyle comprenant de 1 à 12 atomes de carbone, tel qu'un alkyle, un aryle, un aralkyle, un alkaryle ou un cycloalkyle, X est un atome de chlore ou de brome, X étant de préférence un atome de chlore, et m est un nombre compris entre 0 et 2.

De préférence, le dérivé halogéné est choisi parmi le sesquichlorure d'éthylaluminium (Et₃Al₂Cl₃), le dichlorure de méthylaluminium (MeAlCl₂), le dichlorure d'éthylaluminium (EtAlCl₂), le dichlorure d'isobutylaluminium (iBuAlCl₂), le chlorure de diéthylaluminium (Et₂AlCl), pris seul ou en mélange.

Dans certains systèmes catalytiques, les halogénures d'alkyl aluminium peuvent optionnellement être enrichis en trihalogénure d'aluminium de formule AlX₃. Sans que la liste soit limitative, on peut citer à titre d'exemples: le dichloroethylaluminium enrichi avec du trichlorure d'aluminium et ayant par exemple la formule AlEt_{0,9}Cl_{2,1}, le dichloroisobutylaluminium enrichi avec du trichlorure d'aluminium et ayant par exemple la formule AlisBu_{0,9}Cl_{2,1}.

Dans une variante du procédé, le système catalytique peut être mis en oeuvre en milieu biphasique liquide-liquide contenant un milieu à caractère ionique pas ou peu miscible avec la phase organique contenant les produits. Un tel système catalytique est décrit dans le brevet US 5104840B. Dans un tel cas, le milieu à caractère ionique peut comprendre au moins un sel de formule Q⁺A⁻, dans lequel Q⁺ est un cation ammonium ou phosphonium quaternaire ou un mélange des deux ou un cation lithium et A⁻ est un anion coordinant ou non-coordinant de préférence choisi dans le groupe formé par les halogénoaluminates, les organohalogénoaluminates, les organogallates, les organohalogénogallates ou un mélange d'au moins deux de ces composés.

Outre le dérivé halogéné, le système catalytique comprend avantageusement un précurseur de nickel.

Le précurseur de nickel peut être choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, l'octoate de nickel(II), les phénates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de allylnickel(II), le bromure de allylnickel(II), le dimère du chlorure de methallylnickel(II), l'hexafluorophosphate de allylnickel(II), l'hexafluorophosphate de methallylnickel(II), le biscyclopentadienyle de nickel(II), le bisallyl de nickel(II) et le bisméthallyl nickel(II); sous leur forme hydratée ou non, pris seul ou en mélange. Lesdits précurseurs de nickel peuvent éventuellement être complexés à des bases de Lewis.

A la sortie du procédé d'oligomérisation d'oléfines, de préférence du procédé de dimérisation des oléfines, l'effluent réactionnel peut comprendre des oléfines non converties, des alphaoléfines telles que le butène-1, l'hexène-1, l'octène-1, et d'autres produits de réaction de C4 à C10, ainsi qu'un éventuel solvant de la réaction. Ledit effluent comprend aussi au moins en partie du système catalytique utilisé dans la réaction.

Avantageusement selon l'invention, l'effluent de la réaction d'oligomérisation d'oléfines, de préférence de la réaction de dimérisation d'oléfines est mis en contact avec un composé nitrile RC=N comprenant au moins un groupement carbonitrile -C=N, R étant un groupe hydrocarbyle possédant de 1 à 20 atomes de carbone, saturé ou insaturé, linéaire ou ramifié, cyclique ou aromatique, comprenant ou non un hétéroatome. De préférence, le groupe hydrocarbyle R possède de 1 à 15 atomes de carbone, de manière plus préférée de 1 à 10 atomes de carbone. L'hétéroatome est choisi parmi le soufre, l'oxygène, l'azote et le phosphore.

La mise en contact entre l'effluent réactionnel -comprenant le système catalytique comprenant un dérivé halogéné- et le composé nitrile peut être mise en oeuvre directement à la sortie de la réaction d'oligomérisation d'oléfines. Lorsque l'oligomérisation est réalisée en milieu biphasique liquide-liquide contenant un milieu à caractère ionique, la phase organique contenant les oligomères est de préférence séparée de la phase liquide ionique par décantation avant la mise en contact entre l'effluent réactionnel et le composé nitrile.

De préférence, le composé nitrile est choisi parmi l'acétonitrile, le propionitrile, le butanenitrile ou butyronitrile, l'hexanenitrile, le cyclohexanenitrile, l'acrylonitrile, le benzonitrile, le cyclopentanecarbonitrile. De manière plus préférée, le composé nitrile est choisi parmi l'acétonitrile, le propionitrile, le butyronitrile. De manière encore plus préférée, le composé nitrile est l'acétonitrile.

Dans le procédé de neutralisation de l'invention, on mettra en jeu une quantité de composé nitrile suffisante pour éliminer substantiellement tout l'halogène provenant du système catalytique. De préférence, le rapport molaire entre le composé nitrile et le dérivé halogéné est compris entre 1 et 30, de préférence entre 1 et 15, de manière plus préférée entre 1 et 10.

Avantageusement la mise en contact entre l'effluent de la réaction d'oligomérisation d'oléfines, de préférence de la réaction de dimérisation d'oléfines et le composé nitrile est effectuée à une température comprise entre -20°C et 100°C. Avantageusement, la température de neutralisation est celle à laquelle a été conduite la réaction d'oligomérisation d'oléfines, en particulier la réaction de dimérisation d'oléfines.

L'effluent ainsi neutralisé par le procédé de neutralisation selon l'invention pourra éventuellement être ensuite traité à la soude ou à l'eau ou à l'acide, puis soumis à un fractionnement.

### EXEMPLES

Les exemples qui suivent illustrent l'invention sans en limiter la portée.

### Exemple 1 : Effluent sans traitement avec un composé nitrile (non conforme à l'invention)

Dans un ballon, purgé à l'azote sec, on introduit 100 ml d'une coupe octènes issue de la dimérisation des butènes (appelé dimate). Puis on introduit 0,043 % poids (par rapport au dimate) d'octoate de nickel(II), puis 0,18% poids (par rapport au dimate) de dichlorure d'éthylaluminium (EtAlCl₂=EADC). Le système catalytique présente un rapport molaire Al/Ni =15 et comporte 1000 ppm de chlore introduit par l'EADC. Cette solution orangée est chauffée pendant 5 minutes à 50°C sous agitation (2000 tours/minute).

Dans cette solution sont introduits à la seringue, sous agitation, 50 mL de soude aqueuse à 20 % poids préalablement chauffée à 50°C. L'agitation est maintenue pendant 5 minutes puis arrêtée. Les phases organique et aqueuse sont décantées puis la phase aqueuse est soutirée à la canule. 50 mL d'eau, préchauffée à 50°C, sont rajoutés à la phase organique. L'ensemble est agité pendant 5 minutes (2000 tours/minute). La décantation des phases est instantanée. Celles-ci sont limpides et incolores. On réalise deux autres lavages à l'eau. Les phases aqueuses sont soutirées. La phase organique (dimate) est analysée par chromatographie en phase gazeuse (GC) et par fluorescence aux rayons X (FX) pour l'analyse du chlore.

### Exemple 2 : Effluent avec traitement avec un acétonitrile dans un rapport molaire acétonitrile/ EADC = 2,5 (conforme à l'invention)

Dans un ballon, purgé à l'azote sec, on introduit 100 ml d'une coupe octènes issue de la dimérisation des butènes (appelé dimate). Puis on introduit 0,043 % poids (par rapport au dimate) d'octoate de nickel(II), puis 0,18% poids (par rapport au dimate) de dichlorure d'éthylaluminium (EtAlCl₂=EADC). Le système catalytique présente un rapport molaire Al/Ni =15 et comporte 1000 ppm de chlore introduit par l'EADC. Cette solution orangée est chauffée pendant 5 minutes à 50°C sous agitation (2000 tours/minute).

On injecte alors à la seringue de l'acétonitrile (CH₃CN) de la marque LiPerSolv Chromanorm commercialisé chez VWR à 30 ppm H₂O (grade commercial analytique 99,9%) dans un rapport molaire acétonitrile/ EADC = 2,5. On laisse agiter pendant 5 minutes. Il se forme un précipité blanchâtre. On introduit alors la soude comme précédemment. A l'introduction de la soude, le précipité disparait. La procédure est alors identique à celle décrite dans l'exemple 1 avec deux lavages à l'eau. La décantation des phases est instantanée.

### Exemple 3 : Effluent avec traitement avec un acétonitrile dans un rapport molaire acétonitrile/ EADC = 5 (conforme à l'invention)

Cet exemple est réalisé comme l'exemple 2 à ceci près que le rapport molaire acétonitrile/ EADC = 5.

### Exemple 4 : Effluent avec traitement avec un acétonitrile dans un rapport molaire acétonitrile/ EADC = 5 (conforme à l'invention)

Cet exemple est réalisé comme l'exemple 3 à ceci près qu'on utilise une acétonitrile CH₃CN de la marque PanReac de chez Grosseron à 0,1% poids en H₂O (grade commercial analytique 99,7%).

### Exemple 5 : Effluent avec traitement avec un acétonitrile dans un rapport molaire acétonitrile/ EADC = 10 (conforme à l'invention)

Cet exemple est réalisé comme l'exemple 4 à ceci près que le rapport molaire acétonitrile/ EADC = 10.

Le tableau 1 reporte les résultats issus des analyses GC et FX des effluents sans traitement (exemple 1) et des effluents traités selon le procédé de neutralisation selon l'invention.

**Tableau 1 : Analyse des effluents**

| CH₃CN Dimate/EADC | | Analyse GC du dimate (% poids) | | | | Analyse FX du Chlore | |
|---|---|---|---|---|---|---|---|
| | CH₃CN/EADC | %C4 | %C6 | %C8 | %C10+ | ppm Cl | +/-* ppm Cl |
| Dimate départ | | 0,097 | 2,255 | 97,3 | 0,343 | < 5 | |
| Exemple 1 (non conforme) | - | 0,06 | 2,103 | 95,15 | 2,693 | 313 | 8 |
| Exemple 2 (CH₃CN grade 1) | 2,5 | 0,076 | 2,091 | 97,45 | 0,385 | 9 | 2 |
| Exemple 3 (CH₃CN grade 1) | 5 | 0,059 | 1,941 | 97,52 | 0,478 | 7 | 2 |
| Exemple 4 (CH₃CN grade 2) | 5 | 0,078 | 2,115 | 97,46 | 0,345 | 7 | 2 |
| Exemple 5 (CH₃CN grade 2) | 10 | 0,074 | 2,079 | 97,45 | 0,394 | 7 | 2 |

| | | | | | | | |
|---|---|---|---|---|---|---|---|
| *incertitude de mesure | | | | | | | |

La quantité de chlore après traitement selon le procédé de neutralisation à l'acétonitrile selon l'invention est très inférieure à celle obtenue sans traitement.

## Revendications

1. Procédé de neutralisation d'un système catalytique d'oligomérisation d'oléfines, ledit système catalytique comprenant au moins un dérivé halogéné choisi parmi un halogénure d'alkyl aluminium de formule AlR'ₘX₃₋ₘ dans laquelle R' est un radical hydrocarbyle comprenant de 1 à 12 atomes de carbone, X est un atome de chlore ou de brome et m est un nombre compris entre 0 et 2, **caractérisé en ce que** l'on met en contact l'effluent réactionnel comprenant ledit système catalytique avec au moins un composé nitrile RC=N comprenant au moins un groupement carbonitrile -C=N, R étant un groupe hydrocarbyle possédant de 1 à 20 atomes de carbone, saturé ou insaturé, linéaire ou ramifié, cyclique ou aromatique, comprenant ou non un hétéroatome.

2. Procédé selon la revendication 1 dans lequel le groupe hydrocarbyle R possède de 1 à 15 atomes de carbone.

3. Procédé selon la revendication 1 ou 2 dans lequel le composé nitrile est choisi parmi l'acétonitrile, le propionitrile, le butanenitrile ou butyronitrile, l'hexanenitrile, le cyclohexanenitrile, l'acrylonitrile, le benzonitrile, le cyclopentanecarbonitrile.

4. Procédé selon l'une des revendications précédentes dans lequel le rapport molaire entre le composé nitrile et le dérivé halogéné est compris entre 1 et 30.

5. Procédé selon l'une des revendications précédentes dans lequel la mise en contact entre l'effluent de la réaction d'oligomérisation d'oléfines et le composé nitrile est effectuée à une température comprise entre -20°C et 100°C.

6. Procédé selon l'une des revendications précédentes dans lequel la réaction d'oligomérisation des oléfines est un procédé de dimérisation des oléfines.

7. Procédé selon l'une des revendications précédentes dans lequel le dérivé halogéné est choisi parmi le sesquichlorure d'éthylaluminium (Et₃Al₂Cl₃), le dichlorure de méthylaluminium (MeAlCl₂), le dichlorure d'éthylaluminium (EtAlCl₂), le dichlorure d'isobutylaluminium (iBuAlCl₂), le chlorure de diéthylaluminium (Et₂AlCl), pris seul ou en mélange.

8. Procédé selon l'une des revendications précédentes dans lequel les oléfines utilisées comme charge dans le procédé d'oligomérisation d'oléfines ou dans le procédé de dimérisation des oléfines sont des oléfines comportant de 2 à 10 atomes de carbone.
